(19) Europäisches Patentamt
European Patent Office
Office européen des brevets

(11) Numéro de publication : **0 058 330**
**B1**

(12) # FASCICULE DE BREVET EUROPÉEN

(45) Date de publication du fascicule du brevet : **13.06.84**

(21) Numéro de dépôt : **82100682.2**

(22) Date de dépôt : **01.02.82**

(51) Int. Cl.³ : **C 07 C 57/12**, C 07 C 51/41, C 01 F 5/24, C 01 G 49/00, C 10 L 10/02, C 10 L 10/04

(54) **Solutions organiques de complexes ferro-magnésiens à forte teneur en métal et leurs applications.**

(30) Priorité : **13.02.81 FR 8102830**

(43) Date de publication de la demande : **25.08.82 Bulletin 82/34**

(45) Mention de la délivrance du brevet : **13.06.84 Bulletin 84/24**

(84) Etats contractants désignés : **AT BE CH DE GB IT LI LU NL SE**

(56) Documents cités : **FR-A- 2 363 540**

(73) Titulaire : **Sté ELF-FRANCE**
**137, rue de l'Université**
**F-75007 Paris (FR)**

(72) Inventeur : **Faure, Alain**
**Place de l'Ile de France**
**F-42400 Saint Chamond (FR)**
Inventeur : **Gagneux, Joelle**
**29 A. Chemin de Montribould**
**F-69000 Lyon (FR)**

(74) Mandataire : **Cavrois, Philippe**
**Société Nationale ELF-Aquitaine Division Propriété Industrielle Tour Aquitaine, Cedex No 4**
**F-92080 Paris La Défense (FR)**

**EP 0 058 330 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La combustion des fuels, et spécialement des fuels lourds n° 2 de viscosité inférieure à 40 cSt à 100 °C et une teneur en soufre au plus égale à 4 %, pose de plus en plus de problèmes puisque ces combustibles liquides sont fréquemment préparés à partir de résidus très visqueux souvent craqués thermiquement ou catalytiquement, riches en soufre, sodium et vanadium.

Les principaux problèmes rencontrés sont les suivants :

— combustion incomplète : imbrûlés solides et fumerons acides,

— dépôts et corrosion à haute température liée à la présence du soufre et du vanadium, catalyseur d'oxydation de $SO_2$ en $SO_3$.

Ces différents problèmes peuvent être résolus par l'emploi d'additifs organométalliques spécifiques. Par exemple, les catalyseurs de combustion permettent de réduire de façon notable les imbrûlés solides, mais n'ont aucune action sur les corrosions haute et basse températures et les dépôts vanado sulfuriques. Les plus connus sont à base de manganèse, de fer, de calcium et de baryum.

Les additifs agissant sur les corrosions et modifiant la nature des dépôts contiennent essentiellement du magnésium qui peut être utilisé sous la forme solide (oxyde ou hydroxyde), sous forme de suspension dans un solvant organique ou sous la forme oléo-soluble. Cette dernière forme est de loin la plus facilement applicable puisqu'il suffit de diluer l'additif dans le fuel, mais conduit à des coûts relativement élevés.

L'objet de la présente invention concerne des solutions organiques de complexes ferro-magnésiens à forte teneur en métal. Ces additifs nouveaux permettent de résoudre efficacement l'ensemble des problèmes cités précédemment tout en possédant un coût de revient assez faible. En effet, si de nombreuses méthodes de l'art antérieur permettent de fabriquer des sels de fer à un coût modéré, la préparation des sels de magnésium organo-solubles est réalisée par des méthodes délicates et donc onéreuses faisant appel à la technique dite de surbasage. Cette technique consiste à introduire une quantité de base supérieure à la stœchiométrie en formant à l'aide de gaz carbonique des complexes du type :

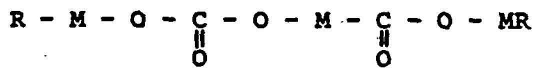

dans lesquels R est un acide sulfonique ou un acide gras par exemple et M = Mg, Ba ou Ca.

La présente invention a pour objet des solutions organiques de nouveaux complexes ferro-magnésiens possédant une forte teneur en métal et le mode de préparation de façon simple et peu coûteuse de ces nouveaux composés dans lesquels les deux métaux actifs sont étroitement combinés au sein d'un même complexe et leur application en tant qu'additifs de combustion multifonctionnels.

Le mode d'obtention de ces solutions organiques de complexes ferro-magnésiens peut être décrit par le procédé type suivant : à un milieu biphasique constitué par une solution d'acide organique dans un solvant organique et d'une solution aqueuse d'un sel ferrique hydrosoluble et d'un sel de magnésium hydrosoluble, est ajoutée, sous agitation, une solution aqueuse concentrée d'une base minérale en quantité telle que le rapport molaire base-sel de magnésium soit compris entre (2 + 3 n) et (3 + 4,5 n) ; n étant le rapport molaire sel de fer-sel de magnésium qui varie de 0,25 à 10. Ceci peut s'expliquer de la manière suivante :

$$FeCl_3 + 3 \text{ mol de base} \rightarrow Fe(OH)_3$$

$$MgCl_2 + 2 \text{ mol de base} \rightarrow Mg(OH)_2$$

on doit donc ajouter 3 moles de base pour 1 Fe et 2 moles de base pour 1 Mg. Pour x moles de fer et y moles de magnésium on doit ajouter au moins 3 x + 2 y moles de base et au plus 4,5 x + 3 y moles de base.

Si $n = xy$

$$Base/Mg = 3\,(x/y) + 2 = 3\,n + 2 \text{ au moins}$$

$$Base/Mg = 4{,}5\,(x/y) + 3 = 4{,}5\,n + 3 \text{ au plus}$$

De façon similaire, le rapport molaire acide organique (sel de magnésium + sel de fer) est compris entre 0,1 et 0,5. Le volume de solvant organique utilisé est directement lié à la teneur en métal de la solution du complexe ferro-magnésien désiré. Après addition totale de la base, le milieu réactionnel est porté à une température comprise entre 60 et 95 °C pendant un temps compris entre 30 mn et 2 h. Un composé du type éther-alcool est alors ajouté en quantité telle que le rapport molaire composé éther-alcool/sels métalliques soit compris entre 0,1 et 1. Après simple homogénéisation à une température

comprise entre 60 et 95 °C, le mélange décante très rapidement en deux phases : solution organique du complexe ferro-magnésien et solution aqueuse des sels minéraux résiduels qui sont séparés.

Selon un autre mode de réalisation préféré de l'invention, au moins la moitié du composé du type alcool éther peut être introduite en même temps que l'acide organique, ce qui permet de réduire la viscosité du milieu réactionnel et d'améliorer la vitesse de réaction des produits.

Conformément à la présente invention, l'acide organique utilisé comprend de 7 à 25 atomes de carbone et est choisi parmi les acides dits « acide gras ». L'agent co-complexant du type éther-alcool est choisi parmi les mono-éthers méthyllique, éthylique, butylique, amylique ou allylique de mono, di ou triéthylène ou propylène glycol. Le sel de fer hydrosoluble est de préférence le chlorure ferrique qui possède une bonne solubilité dans l'eau, mais tout autre sel de fer hydrosoluble convient très bien à la préparation des produits de la présente invention. Le sel de magnésium hydrosoluble est de préférence le chlorure de magnésium. La base minérale peut être la soude, la potasse ou l'ammoniaque mais la base préférée de l'invention est l'ammoniaque qui permet la précipitation totale du magnésium sous forme d'hydroxyde et facilite la formation lors de la coprécipitation des deux hydroxydes métalliques d'un complexe d'insertion ferro-magnésium suffisamment basique pour réagir sur l'acide gras présent dans la phase organique et conduire à une structure complexe pouvant être schématisée par la formule suivante :

$$HO - \overset{X}{\underset{|}{M}} \left[ - O - \overset{X}{\underset{|}{M}} - O - \overset{X}{\underset{|}{M}} \right]_m O - \overset{X}{\underset{|}{M}} - OH$$

M = Fe ou Mg
X = OH ou $RCO_2$ quand M = Fe ($R = C_7$ à $C_{25}$)
$1 \leqq m$

Le choix du solvant utilisé lors de la préparation de ces solutions de complexes ferriques est de préférence guidé par le coût dudit solvant et par les propriétés physiques de la solution de complexes ferro-magnésiens (par exemple, point éclair, viscosité). Il est de préférence choisi parmi différentes coupes pétrolières facilement incorparables aux combustibles liquides : kérosène, résidu de craking catalytique (light cycle oil), gasoil moteur, fuel domestique ou solvants usuels de préférence aromatiques qui conduisent à des solutions peu visqueuses et possédant de très bonnes propriétés à froid (point d'écoulement $< 15$ °C).

La stabilité de ces solutions de complexes ferro-magnésiens est excellente et permet un stockage prolongé sans décantation de composés minéraux. La complexation des ions ferriques et magnésiens présente de plus l'avantage de fournir des solutions organiques peu visqueuses, très facilement diluables par les combustibles liquides à traiter (fuels lourds ; fuel domestique). Les mélanges combustibles liquides sont donc très homogènes, ce qui facilite grandement la mise en œuvre de ce type de solution pouvant contenir de 4 à 12 % de fer et de 0,5 à 9 % de magnésium.

Ces solutions sont incorporées à des combustibles liquides du type fuel domestique ou lourd à des doses telles que la concentration en fer dans le combustible soit comprise entre 5 et 100 ppm et la concentration en magnésium entre 5 et 80 ppm.

Les combustibles liquides ainsi obtenus présentent des propriétés de combustion nettement améliorées : l'émission de suies et d'imbrûlés solides est fortement réduite, pouvant permettre dans certains cas de réduire de façon significative l'excès d'air nécessaire à une bonne combustion ; la nature des dépôts vanadosodiques est fortement modifiée et transformée en dépôts non fusibles limitant ainsi les corrosions haute température et améliorant l'échange thermique ; le pH des fumées est augmenté et la corrosion basse température est fortement limitée.

De plus l'activité de ces additifs ne décroît pas avec l'augmentation de la teneur en imbrûlés, de la teneur en vanadium et en sodium du fuel non additivé. Cet effet est particulièrement intéressant dans le cas des fuels lourds qui du fait de l'alourdissement des bruts et des nouveaux procédés de fabrication (viscoréduction par ex.) conduisent, lors de leur combustion à des teneurs en imbrûlés solides, en fumerons acides et à des corrosions plus importantes que par le passé.

La présente invention est illustrée par les exemples non limitatifs suivants :

## Exemple 1

50 g d'acide oléïque technique (indice d'acide 195, indice d'iode = 90) sont dissous dans 372,5 g de gasoil, 166 g d'une solution à 41 % de chlorure ferrique et 85,5 g de chlorure de magnésium hexahydraté dissous dans 277 g d'eau sont ajoutés à froid sous agitation. A ce mélange, 245 g d'ammoniaque à 22° baumé sont additionnés lentement. Après addition totale, le mélange réactionnel est chauffé sous agitation pendant 1 h 30 à 95° et 16 g de butyldiglycol sont ajoutés. Après 15 mn, l'agitation est arrêtée et le mélange est décanté à chaud et séché par chauffage sous léger vide pour éliminer les dernières traces d'eau. Après filtration, 468 g d'une solution contenant 4,9 % de fer et 2 % de magnésium, et possédant une viscosité cinématique des 5,1 cSt à 100 °C sont obtenus.

Exemple 2

A 150 g d'acide de TALL à 80 % résinique, dissous dans 425 g de Solgil 710, solvant aromatique fabriqué par la Société Rhône Poulenc, constitué de dialkylbenzènes en $C_{12}$, on ajoute 396 g d'une solution de chlorure ferrique à 41 % et 150 g de chlorure de magnésium hexahydraté dissous dans 750 g d'eau.

510 g d'ammoniaque à 22 °C Baumé sont additionnés lentement au mélange fortement agité. Après 1 h de chauffage à 90 °C, l'addition de 66 g de butyldiglycol provoque une décantation immédiate. Après décantation, la phase organique séchée par filtration coalescente contient 9,3 % de fer et 2,3 % de magnésium.

Exemple 3

A 1 875 g d'acide oléïque de l'exemple 1 dissous dans 360 g de SHELL SOL R, solvant aromatique monoalkylbenzène de la société SHELL, on ajoute 396 g de chlorure de magnésium hexahydraté en solution dans 600 g d'eau. 750 g d'ammoniaque à 22° Baumé et 65 g de butyl glycol sont additionnés lentement de telle sorte que le mélange reste suffisamment fluide pour être agité. Lorsque la viscosité est trop importante, le milieu est chauffé progressivement jusqu'à 90 °C et maintenu à cette température pendant 2 h. Au cours de cette étape, l'eau est relarguée progressivement de l'émulsion initialement formée. Après décantation, le mélange organique est filtré et séché par distillation azéotropique ou par coalescence. Un additif possédant une viscosité cinématique de 9,1 cSt à 100 °C et une teneur en fer de 9,6 % et de magnésium de 6,1 % est ainsi obtenu.

Exemple 4

Si dans l'exemple 2, toutes choses étant égale par ailleurs, on remplace le butyldiglycol par du méthylcellosolve et que l'on réalise son introduction de façon identique à celle du butylglycol dans l'exemple 3, on obtient une phase organique plus fluide (9,7 cSt à 100° au lieu de 8,2 cSt à 100 °C) contenant la même teneur en fer et en magnésium.

**Revendications**

1. Complexes ferro-magnésiens solubles dans les solvants organiques utilisés notamment comme additifs de combustion des combustibles liquides, caractérisés en ce qu'ils sont constitués par des sels complexes dérivés d'un acide organique à chaîne aliphatique contenant de 7 à 25 atomes de carbone, de fer ferrique et de magnésium liés chimiquement au sein d'une même molécule selon la formule :

$$HO-\overset{\overset{X}{|}}{M}\left[-O-\overset{\overset{X}{|}}{M}-O-\overset{\overset{X}{|}}{M}\right]_m O-\overset{\overset{X}{|}}{M}-OH$$

dans laquelle

M est le fer ou le magnésium

X représente les groupes OH ou RCOO

m est un nombre entier au moins égal à 1

R = radical aliphatique en $C_7$ à $C_{25}$

2. Complexes ferro-magnésiens selon la revendication 1 caractérisés en ce que le taux de fer est compris entre 4 et 12 % en poids et le taux de magnésium entre 0,5 et 9 % en poids.

3. Procédé de fabrication des complexes ferro-magnésiens selon les revendications 1 et 2 prises dans leur ensemble caractérisé en ce qu'il consiste à faire réagir à 90 °C sous agitation pendant 1 h à 2 h, en milieu biphasique eau-solvant organique, l'acide carboxylique, les sels hydrosolubles de magnésium et de fer et une base minérale en quantité telles que le rapport molaire : acide organique/sel de magnésium + sel de fer soit compris entre 0,1 et 0,5, le rapport molaire : base/sel de magnésium soit compris entre 3 n + 2 et 4,5 n + 3 et le rapport molaire n sel de fer/sel de magnésium soit compris entre 0,25 et 10, la précipitation des complexes étant provoquée par l'addition d'un composé éther-alcool dans des proportions telles que le rapport molaire composé éther-alcool/sels métalliques soit compris entre 0,1 et 1.

4. Procédé de fabrication de complexes ferro-magnésiens selon les revendications précédentes caractérisé en ce que le composé éther-alcool est choisi parmi les monoéthers méthylique, éthylique, butylique, amylique, allylique de mono, di ou triéthylène et propylène glycols.

5. Procédé de fabrication selon les revendications précédentes caractérisé en ce que la base minérale est la potasse, la soude ou l'ammoniaque.

6. Procédé de fabrication selon les revendications précédentes caractérisé en ce que les sels hydrosolubles de fer et de magnésium sont choisis dans le groupe du chlorure ferrique, du nitrate ferrique et du chlorure de magnésium.

7. Additifs améliorant la combustion des combustibles liquides caractérisés en ce qu'ils sont constitués par une solution organique de complexes ferro-magnésiens définis dans les revendications précédentes et contenant de 40 à 120 g/l de fer soluble et de 5 à 90 g/l de magnésium soluble.

**Claims**

1. Ferro-magnesium complexes which are soluble in organic solvents, especially those used as combustion additives for combustible liquids, characterised in that they are constituted by complex salts derived from an organic acid having an aliphatic chain containing from 7 to 25 carbon atoms, ferric iron and magnesium bonded chemically to the interior of a similar molecule having the formula :

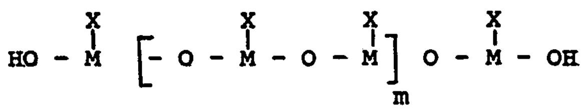

in which

M is iron or magnesium

X represents the groups OH or RCOO

m is an integer at least equal to 1

R = a $C_7$ to $C_{25}$ aliphatic radical.

2. Ferro-magnesium complexes according to claim 1, characterised in that the amount of iron is between 4 and 12 % by weight and the amount of magnesium between 0.5 and 9 % by weight.

3. Process for the preparation of ferro-magnesium complexes according to claims 1 and 2 taken together, characterised in that it consists in reacting the carboxylic acid, the water soluble magnesium and iron salts and a mineral base at 90 °C with agitation for 1 to 2 hours in a 2-phase water-organic solvent medium in quantities such that the molar ratio : organic acid/magnesium salt + iron salt is between 0.1 and 0.5, the molar ratio : base/magnesium salt is between 3 n + 2 and 4.5 n + 3, and the molar ratio n iron salt/magnesium salt is between 0.25 and 10, the precipitation of the complexes being brought about by the addition of an ether-alcohol compound in proportions such that the molar ratio : ether-alcohol compound metallic salts is between 0.1 and 1.

4. Process for the preparation of ferro-magnesium complexes according to the preceding claims, characterised in that the ether-alcohol compound is selected from methyl, ethyl, butyl, amyl or allyl monoethers of mone, di, or triethylene and propylene glycols.

5. Preparative process according to the preceding claims characterised in that the mineral base is potash, soda or ammonia.

6. Preparative process according to the preceding claims characterised in that the water-soluble iron and magnesium salts are selected from the group of ferric chloride, ferric nitrate and magnesium chloride.

7. Additives for improving the combustion of combustible liquids characterised in that they are constituted by an organic solution of ferro-magnesium complexes as defined in the preceding claims and containing from 40 to 120 g/l of soluble iron and from 5 to 90 g/l of soluble magnesium.

**Ansprüche**

1. Eisen- und magnesiumhaltige Komplexe mit Löslichkeit in den organischen Lösemitteln, die insbesondere als Verbrennungszusätze von flüssigen Brennstoffen verwendet werden, dadurch gekennzeichnet, daß sie aus komplexen Salzen bestehen, die abgeleitet sind von einer organischen Säure mit einer 7 bis 25 Kohlenstoffatome enthaltenden aliphatischen Kette, von Eisen (III) und Magnesium, die innerhalb des gleichen Moleküls chemisch gebunden sind, gemäß der Formel

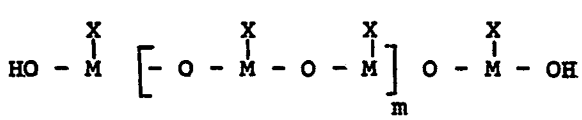

in welcher

M Eisen oder Magnesium

X die Gruppe OH oder RCOO

m eine ganze Zahl von mindestens gleich 1, und

R einen aliphatischen $C_7$-$C_{25}$-Rest bedeuten.

2. Eisen- und magnesiumhaltige Komplexe nach Anspruch 1, dadurch gekennzeichnet, daß der Eisengehalt zwischen 4 und 12 Gew.-% und der Magnesiumgehalt zwischen 0,5 und 9 Gew.-% liegt.

3. Verfahren zur Herstellung von eisen- und magnesiumhaltigen Komplexen nach den Ansprüchen 1 und 2, dadurch gekennzeichnet, daß man unter Rühren in einem zweiphasigen Medium aus Wasser/organischem Lösemittel bei 90 °C während eines Zeitraums von 1 h bis 2 h die Carbonsäure, die wasserlöslichen Salze des Magnesiums und Eisens und eine mineralische Base in einer solchen Menge umsetzen läßt, daß das Molverhältnis von organischer Säure zu Magnesiumsalz plus Eisensalz zwischen 0,1 und 0,5, das Molverhältnis von Base zu Magnesiumsalz zwischen (3 n + 2) und (4,5 n + 3) und das Molverhältnis « n » von Eisensalz zu Magnesiumsalz zwischen 0,25 und 10 liegt, und man die Fällung der Komplexe durch Zugabe einer Etheralkohol-Verbindung in solchen Anteilen, daß das Molverhältnis von Etheralkohol-Verbindung zu Metallsalzen zwischen 0,1 und 1 liegt, bewirkt.

4. Verfahren zur Herstellung von eisen- und magnesiumhaltigen Komplexen nach den vorstehenden Ansprüchen, dadurch gekennzeichnet, daß die Etheralkohol-Verbindung ausgewählt ist aus den Monomethyl-, Monoethyl-, Monobutyl-, Monoamyl- und Monoallylethern des Mono-, Di- oder Triethylenglykols und -propylenglykols.

5. Verfahren zur Herstellung nach den vorstehenden Ansprüchen, dadurch gekennzeichnet, daß die mineralische Base Kalilauge, Natronlauge oder Ammoniak ist.

6. Verfahren zur Herstellung nach den vorstehenden Ansprüchen, dadurch gekennzeichnet, daß die wasserlöslichen Salze des Eisens und Magnesiums ausgewählt sind aus der Gruppe : Eisen(III)chlorid, Eisen(III)-nitrat und Magnesiumchlorid.

7. Zusätze zum Verbessern der Verbrennung von flüssigen Brennstoffen, dadurch gekennzeichnet, daß sie aus einer organischen Lösung von eisen- und magnesiumhaltigen Komplexen der vorstehenden Ansprüche bestehen und 40 bis 120 g/l lösliches Eisen und 5 bis 90 g/l lösliches Magnesium enthalten.